# EUROPÄISCHE PATENTSCHRIFT

(11) **EP 0 848 759 B1**
(45) Veröffentlichungstag und Bekanntmachung des Hinweises auf die Patenterteilung: **31.10.2001**
(21) Anmeldenummer: 96943850.6
(22) Anmeldetag: 05.09.1996
(51) Int. Cl.: C12P 7/62

(54) **EXTRAKTIONSMITTEL FÜR POLYHYDROXYALKANSÄUREN**
AGENTS FOR EXTRACTING POLYHYDROXYALKANE ACIDS
AGENT D'EXTRACTION POUR LES ACIDES DE POLYHYDROXYALCANES

(30) Priorität: 09.09.1995 DE 19533459; 14.06.1996 DE 19623778
(43) Veröffentlichungstag der Anmeldung: 24.06.1998
(73) Patentinhaber: Buna Sow Leuna Olefinverbund GmbH, 06258 Schkopau (DE)
(72) Erfinder: METZNER, Klaus, D-06124 Halle (DE); SELA, Marion, D-06128 Halle (DE); SCHAFFER, Jürgen, D-06122 Halle (DE)
(86) Internationale Anmeldenummer: DE9601660
(87) Internationale Veröffentlichungsnummer: WO9708931

(56) Entgegenhaltungen:
- EP-A- 0 355 307
- WO-A-93/11656
- WO-A-93/23554
- DE-A- 4 215 864
- GB-A- 687 481
- US-A- 4 140 741

## Beschreibung

Die Erfindung betrifft die Verwendung von Extraktionsmitteln zur Gewinnung von Homo- oder Copolymerisaten der Hydroxyalkansäuren aus solchen Stoffen, welche diese Hydroxyalkansäuren enthalten oder zur Gewinnung von Homo- oder Copolymerisaten der Hydroxyalkansäuren in ihrer Form aus verunreinigten Polyhydroxyalkansäuren oder deren Recyclaten.

Verschiedene Mikroorganismen bilden unter bestimmten Fermentationsbedingungen polymere Hydroxyalkansäuren, wie zum Beispiel Poly-β-Hydroxybuttersäure oder Polyhydroxyvaleriansäure. Zur Gewinnung dieser Polyester aus der Bakterienbiomasse sind verschiedene Verfahren der Extraktion mit Lösungsmitteln bekannt, die als spezifische Lösungsmittel chlorierte Kohlenwasserstoffe, wie Chloroform (US-PS 3275610) oder 1,2-Dichlorethan (EP-PS 14490, EP-PS 15123), deren Mischung mit einem Alkohol (US-PS 3044942) oder Pyridin (US-PS 3036959) einsetzen. Nach DD 229428 A 1 kommt Essigsäureanhydrid als Extraktionsmittel für die Gewinnung von Poly-β-Hydroxybuttersäure zur Anwendung, jedoch mit dem Nachteil eines nicht vollständig von Restgerüchen freien Polyesters.

In US-PS 4101533 werden cyclische Kohlensäureester wie Ethylen- oder Propylencarbonat als Lösungsmittel für Poly-β-Hydroxybuttersäure vorgeschlagen. Da diese Extraktionsmittel jedoch in heißem Zustand eine erhöhte Aggressivität aufweisen, ist ein gefahrloser Einsatz nicht in jedem Fall gewährleistet.

In der Patentschrift DD 239609 A1 wird zur Gewinnung von Poly-β-Hydroxybuttersäure aus getrockneter Bakterienbiomasse als Extraktionsmittel eine Mischung aus 1,2-Dichlorethan und Methanol im Verhältnis 1 : 5 bis 1 : 30 eingesetzt, wobei die Extraktionstemperaturen zwischen 40 °C und 75 °C betragen.

In einem Temperaturbereich der Extraktion zwischen 100 °C bis 150 °C stellt die EP-PS 0355 307 B 1 ein Verfahren zur Extraktion von Poly-β-Hydroxybuttersäure vor, bei welchem Diole, acetalisierte Triole, Di- oder Tricarbonsäureester, deren Gemische oder β -Butyrolacton zum Einsatz kommen.

Dem überwiegenden Teil der vorgeschlagenen Extraktionsmittel ist jedoch nachteilig, daß durch eine notwendige lange Extraktionszeit eine Depolymerisierung der Polyhydroxyalkansäuren auftritt, so daß bei diesen Methoden entweder eine schlechte Ausbeute oder ein Abbau des Molekulargewichtes der Polyhydroxyalkansäuren in Kauf genommen werden muß. Andererseits zeigt sich auch bei den angewendeten hohen Extraktionstemperaturen zwischen 100 °C und 150 °C ein Abbau des Molekulargewichtes, was für die Anwendung der Polyhydroxyalkansäuren oder deren Copolymere als Thermoplast nachteilige Folgen hat.

Der Erfindung liegt die Aufgabe zugrunde, für die Gewinnung von Homo- oder Copolymerisaten der Hydroxyalkansäuren ein mit Wasser mischbares Extraktionsmittel vorzuschlagen, welches das Molekulargewicht des Polymeren während des Behandlungsprozesses wenig beeinflußt und physiologisch unbedenklich ist.

Erfindungsgemäß wird die Aufgabe dadurch gelöst, daß Methyllactat oder Ethyllactat als Extraktionsmittel eingesetzt wird. Das Herauslösen von Polyhydroxyalkansäuren kann entweder aus dem Zellmaterial des Mikroorganismus direkt oder nach vorherigem Aufschluß der Zellwände entsprechend bekannter Verfahren erfolgen. Es ist aber auch möglich, eine Verunreinigungen enthaltende Polyhydroxyalkansäure mit dem Extraktionsmittel Methyllactat oder Ethyllactat zu behandeln, um nach den bekannten Verfahrensschritten der Umfällung polymere Hydroxyalkansäuren zu erhalten.

Mit dem erfindungsgemäßen Extraktionsmittel ist aber auch die Möglichkeit gegeben, aus einem im Produktionsprozeß der Polyhydroxyalkansäuren anfallenden Abfall oder aus einem zur Wiederverarbeitung bereitgestellten Recyclat ein Homo- oder Copolymeres der Hydroxyalkansäuren in reiner Form zu gewinnen.

Solche in Methyllactat oder Ethyllactat löslichen polymeren Hydroxyalkansäuren können Poly-β-Hydroxybuttersäure, Polyhydroxyvaleriansäure oder auch Polyhydroxypropionsäure sein sowie deren Copolymere. Dabei ist insbesondere das Copolymere von Polyhydroxybuttersäure und Polyhydroxyvaleriansäure von wirtschaftlichem Interesse.

Zur Gewinnung von reinen polymeren Hydroxyalkansäuren aus Zellmaterial von Mikroorganismen oder aus verunreinigten Polyhydroxyalkansäuren werden die bekannten Extraktionsverfahren angewendet, wobei das polymere Material vorher einer Trocknung unterzogen wird. Eine noch vorhandene Restfeuchte führt bei der Extraktion zu einem erhöhten Abbau des Molekulargewichtes.

Die Extraktion mit dem erfindungsgemäßen Lösungsmittel Methyllactat erfolgt bei dessen Siedetemperatur, vorteilhaft jedoch bei Temperaturen von 100 bis 110 °C, und die Extraktion mit Ethyllactat erfolgt bei einer Siedetemperatur von 154 °C. Das Ausfällen der polymeren Hydroxyalkansäuren kann mit Wasser erfolgen. Der größte Teil der Extraktionsmittel nach dem Stand der Technik ist jedoch mit Wasser nicht mischbar. Das Extraktionsverfahren unter Verwendung von Methyllactat oder Ethyllactat gestattet jedoch ein problemloses Ausfällen der polymeren Hydroxyalkansäuren aus deren Lösung mit Wasser ohne Auftreten von Mischungslücken.

Es sind aber auch andere aus dem Stand der Technik bekannte Fällmittel, wie Methanol oder niedrigsiedende Kohlenwasserstoffe wegen ihrer Mischbarkeit mit den Extraktionsmitteln Methyllactat oder Ethyllactat einsetzbar.

Vorteilhaft kann das gelöste Polymer auch dadurch aus der beschriebenen Lösung gewonnen werden, indem diese auf tiefere Temperaturen, vorzugsweise auf 20 bis 30 °C abgekühlt wird, aus dem entstandenen Gel die Hauptmenge des Lösungsmittels durch mechanisches Abpressen entfernt wird und der verbleibende Lösungsmittelrest mit Wasser oder einem anderen mit den beschriebenen Lösungsmittel mischbaren Fällmittel, z. B. Methanol ausgewaschen wird.

Die erfindungsgemäß verwendeten Lösungsmittel Methyllactat oder Ethyllactat sind physiologisch in hohem Maße für Mensch und Tier verträglich und als Zusatzstoffe für Lebensmittel unbedenklich. Somit haben die nach dem Behandlungsverfahren der Extraktion möglicherweise im Polymerstoff zurückbleibenden Lösungsmittelspuren bei den potentiellen Verwendungen keine störende Wirkung.

Die Erfindung wird an folgenden Beispielen näher erläutert.
Unter Fermentationsbedingungen wurde der Mikroorganismus Methylobacterium rhodesianum kultiviert. Die sprühgetrocknete Biomasse mit einem Gehalt von 35 % Polyhydroxybuttersäure (bezogen auf Feststoff) bei einer durchschnittlichen Molmasse von 250 000 D wurde mit Methyllactat im Verhältnis 1 : 10 30 min bei 135 °C am Rückfluß gekocht. Nach diesem Zeitraum waren 85 % der Polyhydroxybuttersäure aus der Biomasse herausgelöst. Durch Ausfällen in Wasser konnte ein kurzfasriger Niederschlag aus reiner Polyhydroxybuttersäure erhalten werden, deren durchschnittliche Molmasse bei 200 000 D lag.

Sprühgetrocknete Biomasse vom Mikroorganismus Alcaligenes Eutrophus mit einem Gehalt von 60 % Polyhydroxybuttersäure (bezogen auf Feststoff) bei einer durchschnittlichen Molmasse von 450 000 D wurde mit Methyllactat im Verhältnis 1:10 30 Minuten bei 105 °C gekocht. Die Restfeuchte in der Biomasse wurde durch eine Vorextraktion mit Methanol, bei 50 °C über einen Zeitraum von 2 Stunden und anschließender gründlicher Entfernung von Metha - nolspuren, minimiert. Bei der Extraktion mit Methyllactat bei 105 °C war nach einem Zeitraum von 30 Minuten 85 % der Polyhydroxybuttersäure aus der Biomasse herausgelöst. Durch Ausfällen in Wasser konnte ein kurzfasriger Niederschlag aus reiner Polyhydroxybuttersäure erhalten werden, deren durchschnittliche Molmasse bei 350 000 D lag.

Die Lösung von Polyhydroxybuttersäure in Methyllactat wurde bei einer Temperatur oberhalb 70 °C in ein großflächiges, planes Gefäß gegossen und auf Raumtemperatur abgekühlt. Dadurch entstand ein einheitliches und kompaktes Gel. Durch mechanisches Abpressen konnte mindestens die Hälfte des verwendeten Lösungsmittels aus dem Gel entfernt werden. Damit erhöhte sich der Gehalt an Polyhydroxybuttersäure im Gel von 5 % auf 13,5 %. Das restliche Lösungsmittel wurde durch Auswaschen mit Wasser entfernt.

Unter Fermentationsbedingungen wurde der Mikroorganismus Methylobacterium rhodesianum kultiviert. Die sprühgetrocknete Biomasse mit einem Gehalt von 35 % Polyhydroxybuttersäure (bezogen auf Feststoff) bei einer durchschnittlichen Molmasse von 250 000 D wurde mit Ethyllactat im Verhältnis 1 : 10 30 min bei 154 °C im Rückfluß gekocht. Nach diesem Zeitraum waren 85 % der Polyhydroxybuttersäure aus der Biomasse herausgelöst. Durch Ausfällen in Wasser konnte ein kurzfasriger Niederschlag aus reiner Polyhydroxybuttersäure erhalten werden, deren durchschnittliche Molmasse bei 200 000 D lag.

## Patentansprüche

1. Verwendung von Methyllactat oder Ethyllactat als Extraktionsmittel zur Gewinnung eines Homo- oder Copolymerisates von Hydroxyalkansäuren aus einem dieses enthaltenden Stoff.

2. Verwendung von Methyllactat oder Ethyllactat nach Anspruch 1 zur Gewinnung von Homo- oder Copolymerisaten von Hydroxy-alkansäuren aus dem Zellmaterial von Mikroorganismen.

## Claims

1. Use of methyl lactate or ethyl lactate as extracting agent to produce a homopolymer or copolymer of hydroxy alkane acids from a substance containing the latter.

2. Use of methyl lactate or ethyl lactate according to claim 1 above to produce homopolymers or copolymers of hydroxy alkane acids from the cell material of microorganisms.

## Revendications

1. Emploi de lactate de méthyle ou de lactate d'éthyle comme produit d'extraction pour extraire un homopolymérisat ou un copolymérisat d'acides hydroxyalcaniques à partir d'une des matières contenant celui-ci.

2. Emploi de lactate de méthyle ou de lactate d'éthyle selon la revendication 1 pour extraire des homopolymérisats ou des copolymérisats d'acides hydroxyalcaniques à partir de la matière cellulaire de micro-organismes.
